# EUROPÄISCHE PATENTSCHRIFT

(11) **EP 0 595 823 B1**
(45) Veröffentlichungstag und Bekanntmachung des Hinweises auf die Patenterteilung: **22.04.1998**
(21) Anmeldenummer: 92911080.7
(22) Anmeldetag: 10.06.1992
(51) Int. Cl.: A61B 17/36, A61F 9/00

(54) **VORRICHTUNG ZUR SCHONENDEN UND EXAKTEN PHOTOABLATION FÜR PHOTOREFRAKTIVE CHIRURGIE**
DEVICE FOR GENTLE AND PRECISE PHOTOABLATION FOR PHOTOREFRACTIVE SURGERY
DISPOSITIF DE PHOTO-ABLATION DOUCE ET PRECISE POUR LA CHIRURGIE PHOTOREFRACTIVE

(30) Priorität: 10.06.1991 DE 4119024
(43) Veröffentlichungstag der Anmeldung: 11.05.1994
(73) Patentinhaber: CHIRON Technolas GmbH Ophthalmologische Systeme, 85609 Dornach (DE)
(72) Erfinder: HOHLA, Kristian, D-8011 Vaterstetten (DE)
(74) Vertreter: VOSSIUS & PARTNER
(86) Internationale Anmeldenummer: DE9200478
(87) Internationale Veröffentlichungsnummer: WO9222255

(56) Entgegenhaltungen:
- WO-A-87/05496
- FR-A- 2 576 780
- HEALTH PHYSICS, Band 40, Nr. 5, Mai 1981, J. TABOADA et al. "Response of the corneal epithelium to KrF Excimer Laser pulses", Seiten 677-683, siehe Fig. 1; Seite 677, rechte Spalte - Seite 678, 1. Absatz.

## Beschreibung

Die Erfindung bezieht sich auf eine Vorrichtung zur Photoablation, insbesondere für die photorefraktive Chirurgie, mit einem gepulsten Lasersystem, dessen Laserstrahl auf einen zu ablatierenden Bereich gerichtet ist.

Vorrichtungen zur Photablation mit einem gepulsten Lasersystem werden derzeit in der Technik und in der Medizin zur Bearbeitung bzw. Behandlung der verschiedenartigsten Materialien eingesetzt:

Beispielsweise bei der lasertherapeutischen photoablativen Chirurgie der Cornea wird mit Hilfe eines Lasers, der in Art einer Fräse im mikrochirurgischen Bereich eingesetzt wird, die Oberfläche der Cornea so bearbeitet bzw. geformt, daß Sehfehler zumindest annähernd korrigiert werden.

Hierzu wird häufig ein Excimerlaser benutzt, der Laserlicht im UV-Bereich emittiert. Ein derartiger Laserstrahl "verdampft" das organische Gewebe "auf kaltem Wege". Hierdurch ist es - theoretisch - möglich, der Cornea die gewünschte Form mit großer Genauigkeit zu geben: Da typischerweise pro Laserpuls - je nach deponierter Energie - Materialschichten mit einer Dicke von weniger als 1 µm abgetragen werden, kann durch Applikation mehrerer Laserpulse auf die gleiche Gewebestelle nahezu jeder gewünschter Gewebeabtrag erfolgen.

Damit lassen sich sowohl Astigmatismus als auch Hyperopien zumindest in gewissen Grenzen beheben.

Die FR-A-2 576 780 beschreibt eine Vorrichtung zur Berichtigung eines Teils der Krümmung der Hornhaut durch Photoablation einer Hornhautzone. Dabei wird ein Laserstrahl mit einer Wellenlänge von etwa 0,2 µm mit einem optischen System auf die zu entfernende Hornhautzone gerichtet und dort auf einem Teil der Zone ein Leuchtfleck gebildet, der beständig auf der optischen Achse des Auges zentriert ist. Durch fortschreitendes Variieren der Fläche des Flecks werden die Teile der zu entfernenden Zone um so länger dem Strahlenbündel ausgesetzt, wie diese der größeren Dicke der abzutragenden Hornhautsubstanz entsprechen.

Die WO-A-8 705 496 betrifft eine Vorrichtung zur Korrektur der Hornhautkrümmung, bei der ein Laserstrahl durch eine erodierbare Maske auf eine abzutragende Hornhautzone geleitet wird. Die erodierbare Maske wird entsprechend der gewünschten zu erzielenden Hornhautabtragung zuvor hergestellt. Beispielsweise wird das Dickenprofil so gewählt, daß ausgehend von der Mitte der erodierbaren Maske ein größer werdendes Loch entsteht, durch das die Laserstrahlen auf der zu behandelnden Zone von der Mitte zum Rand hin einen geringeren Abtrag erzielen.

Daneben wird die photoablative Chirurgie zur Glättung bei Nahtbildungen etc. benutzt.

In der Praxis hat sich jedoch die Applikation des Laserstrahls zur präzisen Ablation als schwierig herausgestellt. Deshalb werden derzeit nur Schichtdicken von ca. 30 bis maximal 50 µm abgetragen; dabei hat sich jedoch herausgestellt, daß bei den bekannten Vorrichtungen, von denen bei der Formulierung des Oberbegriffs des Anspruchs 1 ausgegangen worden ist, sich die Abtragrate über das Sichtfeld der Cornea hinweg stark ändert.

Die Ursache hierfür dürfte unter anderem sein, daß sich die Energie der Laserpulse von Laserpuls zu Laserpuls je nach Lasersystem mehr oder weniger - in Extremfällen bis zu ±30% und mehr - ändert, so daß die "Abtragrate pro Laserimpuls" nicht konstant ist.

Zusätzlich enstehen bei den bekannten Vorrichtungen zur Photoablation, bei denen Laserpulse mit Energiedichten von ca. 120 bis 200 mJ/cm² verwendet werden, Druckwellen, die in das Gewebe hineinlaufen. Diese Wellen können zu einer Schädigung der unterhalb des bearbeiteten Gebietes liegenden Areale führen. Diese Schädigungen machen sich langfristig in einer Trübung der Cornea (Haze-Bildung) bemerkbar, die in der Regel sehr schwach, aber doch bemerkbar ist, und damit zu einer Beeinträchtigung der Sehfähigkeit führt.

Weiterhin besteht bei den bekannten Vorrichtungen zur Photoablation für die photorefraktive Chirurgie folgendes Problem:

Zur Behebung von Augenfehler muß das Gewebe der Cornea großflächig und in unterschiedlichen Bereichen unterschiedlich stark abgetragen werden. Dabei dürfen jedoch zwischen den unterschiedlich stark zu ablatierenden Bereichen keine Stufen auftreten, da dies unerwünschte Effekte auf die Sehkraft haben würde.

Hierzu werden derzeit im wesentlichen folgende Verfahren verwendet:
1. Bei einem Verfahren wird versucht, die Energiedichteschwankungen des Laserstrahl über dessen Strahlfleckflache so gering wie möglich zu halten (im Bereich von wenigen Prozent). Ein derartiger Laserstrahl wird aufgeweitet und durch kreisförmige Blenden begrenzt. Die Blenden sind dabei derart dimensioniert, so daß sie auf der Cornea entsprechend verkleinert abgebildet werden. von Laserpuls zu Laserpuls wird die Blende etwas geschlossen, so daß die weiter außen liegenden Zonen der Cornea weniger stark ablatiert werden als der Zentralbereich.
2. Kann man keine ausreichend kleinen Energiedichteschwankungen des Laserstrahl über dessen Strahlfleckfläche erzielen, so kann man zwar ebenfalls wie unter 1. beschrieben vorgehen, in diesem Falle ist es jedoch erforderlich, den Laserstrahl ständig um die optische Achse beispielsweise mit Hilfe eines Dove-Prismas zu drehen, um die Homogeniätsschwankungen auszugleichen.
3. Anstelle von sich kontinuierlich schließenden Blenden können auch diskrete Blenden mit unterschiedlichem Durchmesser verwendet werden, die beispielsweise in einer sog. Recoss-Scheibe vorgesehen sind. Nacheinander werden die Blenden mit unterschiedlichem Durchmesser in den Strahlengang eingebracht, so daß man unterschiedlich stark ablatierte Zonen erhält.
4. Bei einem weiteren Verfahren wird der Laserstrahl durch eine Zylinderlinse linienförmig in eine Zwischenbildebene abgebildet. Das Zwischenbild wird mit Hilfe eines Scanners über die Cornea geführt wird. Vor der Cornea befindet sich eine Blende, die sich entsprechend der zu behandelnden Fehlsichtigkeit langsam schließt. Bei dieser Methode kann auf eine hohe Homogenität des Strahles verzichtet werden.
5. Darüberhinaus ist vorgeschlagen worden, mit einem homogenen Laserstrahl eine in einem gewissen Abstand vor der Cornea angebrachte dünne Kunststoffscheibe zu beleuchten, die ein spezielles Dickenverhalten aufweist. Wird zum Beispiel eine Folie, die im zentralen Bereich eine geringere Dicke als am Randbereich aufweist, von einem Laserstrahl getroffen, so wird sie zunächst im Zentrum total ablatiert und damit durchsichtig werden. Der transparente Bereich wird sich nach außen hin erweitern, so daß eine sich automatisch öffnende Blendenwirkung entsteht. Das durch diese sich öffnende Blende hindurchtretende Laserlicht wird die Cornea entsprechend formen; d.h. durch eine spezielle Formgebung dieser Blende läßt sich das Negativ hiervon in die Cornea schneiden.

Bei allen vorgenannten Methoden wird jedoch davon ausgegangen, daß sich die Cornea in ihrem Schnitt- bzw. Ablationsverhalten in bekannter Weise verhält, d.h. das Ablationsverhalten sämtlicher zu behandelnder Hornhäute wird als gleich eingestuft. Die erzielten Schnitttiefen und -formen werden dabei nicht aktiv kontrolliert, sondern entsprechend üblicher Erfahrungswerte "geschätzt".

Im übrigen wird bei den bekannten Ablationsverfahren nicht berücksichtigt, daß Energiedichten benutzt werden, durch die in das Gewerbe hineinlaufende Druckwellen entstehen, die zu pathologischen Schädigungen des Gewebes führen können, bis hin zur Beeinträchtigung des Sehvermögens, oder daß die Energie der einzelnen Laserpulse zum Teil beträchtlich schwankt.

Der Erfindung liegt daher die Aufgabe zugrunde, eine Vorrichtung zur Photoablation, insbesondere für die photorefraktive Chirurgie, mit einem gepulsten Lasersystem, dessen Laserstrahl auf einen zu ablatierenden Bereich gerichtet ist, anzugeben, bei der das Ablationsverhalten kontrolliert wird.

Des weiteren sollen schädigende Druckwellen, die durch den Energieeintrag in das Gewebe entstehen können, vermieden werden.

Eine erfindungsgemäße Lösung dieser Aufgabe ist im Anspruch 1 angegeben. Weiterbildungen der Erfindung sind Gegenstand der Unteransprüche.

Erfindungsgemäß ist die Vorrichtung zur Photoablation, insbesondere für die photorefraktive Chirurgie, mit einem gepulsten Lasersystem, dessen Laserstrahl auf einen zu ablatierenden Bereich gerichtet ist, derart ausgebildet, daß im Strahlengang des Lasersystems vor dem zu ablatierenden Bereich ein Strahlteiler angeordnet ist, und daß im Strahlengang des aus dem Strahlteiler austretenden Teilstrahls, der nicht auf den zu ablatierenden Bereich gerichtet ist, ein schichtförmiges Material, dessen Ablationseigenschaft mit dem Material des abzutragenden Bereichs vergleichbar ist, und hinter dem schichtförmigen Material mindestens ein Photosensor vorgesehen sind, dessen Ausgangssignal an eine Steuereinheit angelegt ist, die bei Bestrahlung des Photosensors weitere Laserimpulse unterbindet.

Eine Reduzierung der vorgenannten Druckwellenbildung, die sich während der Energiedeponierung pro Laserimpuls innerhalb der zu behandelnden Bereiche ausbildet und insbesondere bei der Behandlung der Cornea zu irreversiblen Schäden führen kann, läßt sich unmittelbar durch Herabsetzen der Laserpulsenergie erreichen. Die pro Laserschuß applizierte Energie wird deshalb um den Faktor 10 bis 20 herabgesetzt, wodurch auch die Druckwelle entsprechend abgeschwächt wird.

Natürlich ist mit einer geringeren Pulsenergie auch eine kleinere Photoablationsrate verbunden, was sich nachteilhaft auf die Behandlungsdauer auswirkt. Um einer kleiner werdenden Abtragerate vorzubeugen, wird erfindungsgemäß die Wiederholfrequenz des gepulsten Lasersystems auf 100 bis 500 Hz erhöht. Typische Vorrichtungen hingegen mit Repetitionsraten zwischen 10 und 30 Hz.

Hierbei muß berücksichtigt werden, daß der zeitliche Abstand zwischen zwei Laserpulsen derart zu wählen ist, daß die stoßwelle des ersten Laserpulses sich bereits soweit verdünnt hat, daß eine Überlagerung mit der nachfolgenden ausgesendeten Druckwelle ausgeschlossen werden kann. Da sich Druckwellen typischerweise mit einigen 1000 m/sec ausbreiten und die Gewebetiefe, innerhalb der eine Schädigung vermieden werden soll, maximal 1 mm beträgt, berechnet sich so eine minimale Zeit zwischen zwei Pulsen zu ca. 10⁻⁶ Sekunden. Wiederholfrequenzen im Bereich zwischen 10⁻² bis 10⁻³ Sekunden sind daher unkritisch.

Bereits mit einem derartigen Lasersystem, bei dem der Laserstrahl auf eine Fläche mit einer Größe von 0,01 bis 1 mm² fokussiert wird, kann die Cornea mit Hilfe einer zweidimensionalen Scanningeinheit abgetastet und bearbeitet werden. Ferner können die in den vorgenannten Punkten 1 bis 5 beschriebenen Methoden angewendet werden. Nachteilhaft bleibt jedoch, daß der Materialabtrag unkontrolliert vollzogen wird und sich auf bloße Erfahrungswerte stützt.

Zum Unterschied hierzu wird erfindungsgemäß eine Vorrichtung angegeben, mit der der gesamte Ablationsvorgang kontrolliert durchgeführt werden kann und eine unnötige Belastung für das nichtabzutragende Gewebe ausgeschlossen werden kann.

Eine bevorzugte Ausführung der erfindungsgemäßen Vorrichtung wird nachstehend unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigt:

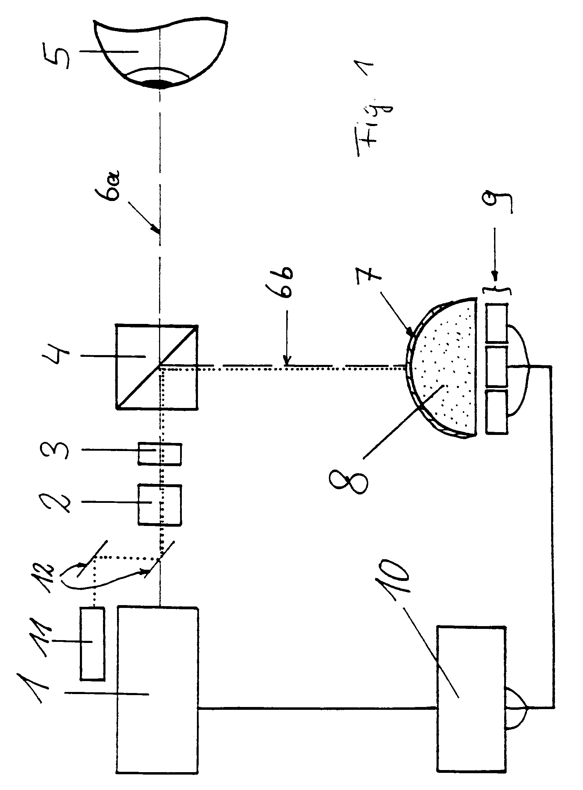

- Figur 1: eine schematische Darstellung aller Vorrichtungskomponenten.

Das in Fig. 1 dargestellte Lasersystem 1, das vorzugsweise aus einem Excimerlaser besteht, erzeugt Lichtimpulse, deren Strahlengang in Figur 1 als gestrichelte Linien dargestellt ist.
Eine zweidimensionale Scanningeinheit 2 sorgt für eine, senkrecht zur Strahlrichtung stehende flächenhafte Ablenkung. Eine Abbildungsoptik 3 stellt den für die Behandlung erforderlichen Strahlquerschnitt ein. Der Laserstrahl wird vor der Cornea 5 mit Hilfe eines Strahlteilers geteilt. Der eine Teil des Laserstrahls 6a tritt durch den Strahlteiler hindurch und trifft auf die Cornea 5, während die andere Hälfte 6b auf ein sogenanntes "Kunstauge" trifft. Das Kunstauge besteht aus einem durchsichtigen Glaskörper 8, auf dessen dem Laserstrahl zugewandten Seite, sich eine dünne Folie 7 befindet. Diese dünne Folie 7 weist in der Größe mindestens eine der abzutragenden Fläche auf der Cornea vergleichbare Fläche auf. Der Laserstrahl wird nun sowohl auf der Cornea 5 als auch auf dem Kunstauge 8, gesteuert durch die Scanning-Einheit 2, im gleichen Rhythmus und in der gleichen Orientierung hin und her bewegt. Hinter dem durchsichtigen Glaskörper 8 befindet sich eine photoempfindliche Diode 9, die auch eine Quadrantenphotodiode oder ein Diodenarray sein kann.

Hat der Laserstrahl die auf dem Glaskörper befindliche Folie an einer Stelle vollkommen abgetragen, so wird die Photodiode 9 belichtet. Das Ausgangssignal der Photodiode 9 liegt an einer Steuereinheit 10 an, die dann, mit dem Lasersystem 1 verbunden, weitere Laserimpulse unterbindet. Auf diese Weise kann erreicht werden, daß der Laserstrahl die Folie 7 an allen Stellen so weit bearbeitet, bis sie vollständig abgetragen wird, ohne daß der Glaskörper selbst von dem Laserstrahl getroffen wird.

Erfindungsgemäß ist es ferner möglich, parallel zu dem Laserstrahl mittels einer Lichtquelle 11 und diversen Einkoppelspiegeln 12 einen kontinuierlichen Hilfslichtstrahl (gestrichelt dargestellt) in den Strahlengang einzukoppeln, dessen Wellenlänge ebenfalls von der auf dem Kunstauge 8 befindlichen Folie 7 absorbiert wird. Sobald der Excimerlaser die dünne Folie 7 "durchgefräst" hat, passiert der Hilfslichtstrahl die Folie 7 und erzeugt das den Laserstrahl unterbindende Signal auf der Photodiode 9.

Die Steuereinheit 10 wird dabei derart betrieben, daß vor jedem Laserimpuls das Ausgangssignal der Photodiode 9 untersucht wird und in Abhängigkeit davon der nächstfolgende Laserschuß ausgelöst oder unterbunden wird. Auf diese Weise läßt sich sicherstellen, daß die gesamte Folie 7 während des Scan-Vorgangs ablatiert wird, ohne dabei den darunterliegenden Glaskörper 8 zu verletzen. Da sich die Folie 7 in ihrem Ablationsverhalten analog zu dem der Cornea 5 verhält, wird auf diese Weise eine Replikation des Folienabtrags auf der Cornea 5 gebildet, d.h. auf der Cornea wird das gleiche Stück abgefräst wie es der abgetragenen Foliendicke und Foliengröße entspricht.

Mit der Verwendung von Quadrantenphotodioden oder Photodiodenarrays ist zusätzlich die Form und Größe der abgetragenen Schicht auf der Cornea meß- und datentechnisch erfaßbar. Hierzu ist zusätzlich eine Auswerte-und Datenspeichereinheit (nicht dargestellt) erforderlich. Somit sind auch Aussagen über die Homogenität des Laserstrahls möglich. Die zur Spezifikation und Herstellung der auf dem Kunstauge aufzutragenden Folie erfolgt entweder nach entsprechenden Erfahrungswerten oder kann exakt der aktuellen Cornea angepaßt werden. Mit Hilfe von Vorversuchen an kleinen Teilen der Cornea läßt sich das genaue Ablationsverhalten und damit die entsprechende Folienkontur exakt bestimmten. Mit Hilfe der erfindungsgemäßen Vorrichtung lassen sich Schwankungen und maschinelle Unsicherheiten auf der Laserseite vollständig ausgleichen, da für den Behandlungserfolg lediglich das den Laserstrahl unterbindende Diodensignal ausschlaggebend ist.

## Patentansprüche

1. Vorrichtung zur Photoablation, insbesondere für die photorefraktive Chirurgie, mit einem gepulsten Lasersystem (1), dessen Laserstrahl auf einen zu ablatierenden Bereich gerichtet ist,
dadurch **gekennzeichnet**, daß im Strahlengang des Lasersystems vor dem zu ablatierenden Bereich ein Strahlteiler (4) angeordnet ist, und
daß im Strahlengang des aus dem strahlteiler austretenden Teilstrahls (66), der nicht auf den zu ablatierenden Bereich gerichtet ist, ein schichtförmiges Material (7), dessen Ablationseigenschaft mit der des Materials des abzutragenden Bereichs vergleichbar ist, und hinter dem schichtförmigen Material mindestens ein Photosensor (9) vorgesehen sind, dessen Ausgangssignal an eine Steuereinheit (10) angelegt ist, die bei Bestrahlung des Photosensors weitere Laserimpulse unterbindet.

2. Vorrichtung nach Anspruch 1,
dadurch **gekennzeichnet**, daß der Materialabtrag durch die auf dem schichtförmigen Material (7) deponierte Laserenergie pro Laserimpuls in einem linearen Verhältnis zum Materialabtrag innerhalb des abzutragenen Bereichs steht.

3. Vorrichtung nach Anspruch 1 oder 2,
dadurch **gekennzeichnet**, daß das schichtförmige Material (7) eine Folie ist.

4. Vorrichtung nach einem der Ansprüche 1 bis 3,
dadurch **gekennzeichnet**, daß die Oberflächengestalt der Folie (7) der äußeren Form des zu behandelnden Gegenstandes (5) ähnlich ist.

5. Vorrichtung nach einem der Ansprüche 1 bis 4,
dadurch **gekennzeichnet**, daß bei der photorefraktiven Chirurgie des Auges die Folie (7) auf einen dem zu behandelnden Auge nachgebildeten, Glaskörper (8) aufgebracht ist.

6. Vorrichtung nach einem der Ansprüche 1 bis 5,
dadurch **gekennzeichnet**, daß die beiden aus dem Strahlteiler austretenden Teilstrahlen (6a, 6b) hinsichtlich ihrer energetischen und geometrischen Eigenschaften weitgehend übereinstimmen.

7. Vorrichtung nach einem der Ansprüche 1 bis 6,
dadurch **gekennzeichnet**, daß die Größe der Fokusfläche der Laserstrahlung zwischen 0,01 und 1 mm² liegt.

8. Vorrichtung nach einem der Ansprüche 1 bis 7,
dadurch **gekennzeichnet**, daß die Wiederholfrequenz des Lasersystems (1) zwischen 100 Hz und 500 Hz liegt.

9. Vorrichtung nach einem der Ansprüche 1 bis 8,
dadurch **gekennzeichnet**, daß parallel zum Laserstrahl ein kontinuierlicher Hilfslichtstrahl geführt ist, dessen Wellenlänge von der Folie absorbiert wird.

10. Vorrichtung nach einem der Ansprüche 1 bis 9,
dadurch **gekennzeichnet**, daß die Steuereinheit (10) vor jedem Laserschuß das Ausgangssignal des Photosensors (9) auswertet und in Abhängigkeit davon den nächstfolgenden Laserschuß ansteuert.

11. Vorrichtung nach einem der Ansprüche 1 bis 10,
dadurch **gekennzeichnet**, daß die Größe und Gestalt des durch den Laser abgetragenen Bereichs in der Folie (7) mit der Größe und Gestalt des abgetragenen Materialbereichs des zu behandelnden Gegenstands (5) in einem linearen Zusammenhang steht.

12. Vorrichtung nach einem der Ansprüche 1 bis 11,
dadurch **gekennzeichnet**, daß das Ablationsverhalten der Folie (7) durch Vorversuche an kleinen Bereichen des abzutragenden Materials bestimmbar ist.

13. Vorrichtung nach einem der Ansprüche 1 bis 12,
dadurch **gekennzeichnet**, daß in einem der beiden Strahlengänge (6a, 6b) ein variabler optischer Abschwächer angebracht ist, der den Strahl so abschwächt, daß das ablative Verhalten des schichtförmigen Materials (7) gleich dem des abzutragenen Materials (5) ist.

14. Vorrichtung nach einem der Ansprüche 1 bis 13,
dadurch **gekennzeichnet**, daß der Photosensor (9) eine Quadrantenphotodiode ist.

15. Vorrichtung nach einem der Ansprüche 1 bis 14,
dadurch **gekennzeichnet**, daß der Photosensor (9) ein Photodiodenarray ist.

## Claims

1. Device for photoablation, in particular for photorefractive surgery, having a pulsed laser system (1) whose laser beam is directed to a zone to be ablated,
characterized in that a beam splitter (4) is arranged in the beam path of the laser system in front of the zone to be ablated, and
that a sheet-like material (7) whose ablation properties are comparable with those of the material of the zone to be removed is provided in the beam path of the partial beam (6b) which emerges from the beam splitter and is not directed to the zone to be ablated, and behind the sheet-like material at least one photosensor (9) is provided, whose output signal is fed to a control unit (10) which prevents further laser pulses when the photosensor is exposed to radiation.

2. The device according to claim 1, characterized in that the material removed by the laser energy applied on the sheet-like material (7) per laser pulse is in a linear relationship to the material removed within the zone to be removed.

3. The device according to claim 1 or 2, characterized in that the sheet-like material (7) is a sheeting.

4. The device according to any one of claims 1 to 3, characterized in that the surface shape of the sheeting (7) is similar to the external shape of the object (5) to be treated.

5. The device according to any one of claims 1 to 4, characterized in that in the photorefractive surgery of the eye the sheeting (7) is deposited on a glass body (8) being an imitation of the eye to be treated.

6. The device according to any one of claims 1 to 5, characterized in that the two partial beams (6a, 6b) emerging from the beam splitter are mainly identical with respect to their energetic and geometric properties.

7. The device according to any one of claims 1 to 6, characterized in that the size of the focusing zone of the laser irradiation is between 0.01 and 1 mm².

8. The device according to any one of claims 1 to 7, characterized in that the repetition frequency of the laser system (1) is between 100 Hz and 500 Hz.

9. The device according to any one of claims 1 to 8, characterized in that a continuous auxiliary light beam whose wavelength is absorbed by the sheeting is guided parallel to the laser beam.

10. The device according to any one of claims 1 to 9, characterized in that prior to each laser shot the control unit (10) evaluates the output signal of the photosensor (9) and, depending thereon, triggers the next laser shot.

11. The device according to any one of claims 1 to 10, characterized in that the size and shape of the zone in the sheeting (7) removed by the laser is in a linear relationship to the size and shape of the removed material zone of the object (5) to be treated.

12. The device according to any one of claims 1 to 11, characterized in that the ablation behaviour of the sheeting (7) can be determined by preliminary tests on smaller zones of the material to be removed.

13. The device according to any one of claims 1 to 12, characterized in that in one of the two beam paths (6a, 6b) a variable optical attenuator is arranged which attenuates the beam such that the ablation behaviour of the sheet-like material (7) is equal to that of the material (5) to be removed.

14. The device according to any one of claims 1 to 13, characterized in that the photosensor (9) is a quadrant photodiode.

15. The device according to any one of claims 1 to 14, characterized in that the photosensor (9) is a photodiode array.

## Revendications

1. Dispositif de photoablation, en particulier pour la chirurgie photoréfractive, comprenant un système laser impulsionnel (1) dont le rayon laser est dirigé sur une zone à soumettre à une ablation,
caractérisé en ce qu'un séparateur de faisceau (4) est agencé dans le trajet du rayon du système laser, en amont de la zone à soumettre à une ablation, et en ce que, dans le trajet du rayon partiel (6b) qui est issu du séparateur de faisceau et qui n'est pas dirigé sur la zone à soumettre à une ablation, sont prévus une matière en forme de couche (7), dont la propriété d'ablation est comparable à la matière de la zone à éliminer, et au moins un photodétecteur (9) derrière la matière en forme de couche, dont le signal de sortie est appliqué à une unité de commande (10) qui fait cesser des impulsions laser supplémentaires lors de l'irradiation du photodétecteur.

2. Dispositif suivant la revendication 1, caractérisé en ce que l'élimination de matière par l'énergie de laser déposée sur la matière en forme de couche (7) par impulsion laser se trouve dans une relation linéaire vis-à-vis de l'élimination de matière à l'intérieur de la zone à enlever.

3. Dispositif suivant l'une des revendications 1 et 2, caractérisé en ce que la matière en forme de couche (7) est une feuille.

4. Dispositif suivant l'une des revendications 1 à 3, caractérisé en ce que la conformation de surface de la feuille (7) est analogue à la forme extérieure de l'objet à traiter (5).

5. Dispositif suivant l'une des revendications 1 à 4, caractérisé en ce que, dans le cas de la chirurgie photoréfractive de l'oeil, la feuille (7) est appliquée sur un corps de verre (8) imitant l'oeil à traiter.

6. Dispositif suivant l'une des revendications 1 à 5, caractérisé en ce que les deux rayons parties (6a, 6b) sortant du séparateur de faisceau concordent largement en ce qui concerne leurs propriétés énergétiques et géométriques.

7. Dispositif suivant l'une des revendications 1 à 6, caractérisé en ce que la grandeur de la surface focale du rayonnement laser est comprise entre 0,01 et 1 mm².

8. Dispositif suivant l'une des revendications 1 à 7, caractérisé en ce que la fréquence de répétition du système laser (1) est comprise entre 100 Hz et 500 Hz.

9. Dispositif suivant l'une des revendications 1 à 8, caractérisé en ce qu'un rayon lumineux auxiliaire continu, dont la longueur d'onde est absorbée par la feuille, est guidé parallèlement au rayon laser.

10. Dispositif suivant l'une des revendications 1 à 9, caractérisé en ce que l'unité de commande (10) évalue le signal de sortie du photodétecteur (9) avant chaque décharge du laser et, en fonction de cela, commande la décharge suivante.

11. Dispositif suivant l'une des revendications 1 à 10, caractérisé en ce que la grandeur et la conformation de la zone enlevée par le laser dans la feuille (7) est en rapport linéaire avec la grandeur et la conformation de la zone de matière enlevée de l'objet à traiter (5).

12. Dispositif suivant l'une des revendications 1 à 11, caractérisé en ce que le comportement à l'ablation de la feuille (7) peut être déterminé par des essais préalables sur de petites zones de la matière à enlever.

13. Dispositif suivant l'une des revendications 1 à 12, caractérisé en ce que, dans un des deux trajets de rayon (6a, 6b), est agencé un organe d'affaiblissement optique variable qui affaiblit le rayon de façon que le comportement à l'ablation de la matière en forme de couche (7) soit identique à celui de la matière à enlever (5).

14. Dispositif suivant l'une des revendications 1 à 13, caractérisé en ce que le photodétecteur (9) est une photodiode à quadrants.

15. Dispositif suivant l'une des revendications 1 à 14, caractérisé en ce que le photodétecteur (9) est un arrangement de photodiodes.
